(19) Europäisches Patentamt
European Patent Office
Office européen des brevets

(11) Publication number: **0 224 260 A2**

(12) **EUROPEAN PATENT APPLICATION**

(21) Application number: 86116474.7

(22) Date of filing: 27.11.86

(51) Int. Cl.4: C07H 13/06 , A61K 31/70 , A61K 39/39 , C12N 9/00

(30) Priority: 28.11.85 JP 268802/85
24.07.86 JP 174436/86
11.08.86 JP 188215/86

(43) Date of publication of application: 03.06.87 Bulletin 87/23

(84) Designated Contracting States: CH DE FR GB IT LI NL SE

(71) Applicant: Toho Yakuhin Kogyo Kabushiki Kaisha
Yachiyo Building 1-14, Awaji-machi
Higashi-ku Osaka-shi Osaka-fu(JP)

(72) Inventor: Hasegawa, Akira
1735-160, Kano'okurayama
Gifu-shi Gifu-ken(JP)
Inventor: Kiso, Makoto
57-47, Monju, Motosu-cho
Motosu-gun Gifu-ken(JP)
Inventor: Morihara, Kazuyuki
5-9-2, Hirose Shimamoto-Cho
Mishima-gun Osaka-fu(JP)

(74) Representative: Vossius & Partner
Siebertstrasse 4 P.O. Box 86 07 67
D-8000 München 86(DE)

(54) **Analogs of nonreducing monosaccharide moiety of lipid A.**

(57) Provided herein is a derivative of 2-deoxy-2-amino-4-O-phophono-D-glucopyranose, which is derived from lipid A, of the formula:

EP 0 224 260 A2

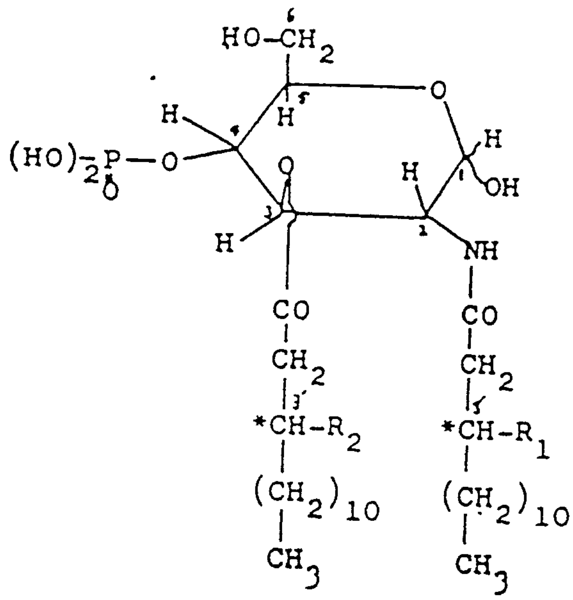

wherein $R_1$ and $R_2$ are a member in a pair selected from the group consisting of those indicated in a following table;

| Compound No. | $R_1$ | $R_2$ |
|---|---|---|
| I(R,R) | $-O-CO-(CH_2)_{12}-CH_3$ | $-O-CO-(CH_2)_{12}-CH_3$ |
| II | $-O-CO-(CH_2)_{12}-CH_3$ | -OH |
| II(R,R) | $-O-CO-(CH_2)_{12}-CH_3$ | -OH |
| II(S,S) | $-O-CO-(CH_2)_{12}-CH_3$ | -OH |
| III | -OH | $-O-CO-(CH_2)_{12}-CH_3$ |
| III(R,R) | -OH | $-O-CO-(CH_2)_{12}-CH_3$ |
| III(S,S) | -OH | $-O-CO-(CH_2)_{12}-CH_3$ |
| IV(R) | -H | $-O-CO-(CH_2)_{12}-CH_3$ |

The compounds of this invention contain ones of a rectus and a sinister configurations and are expected to exhibit more improved biological and immunological activities than those which natural lipid A possesses originally.

# " Analogs of Nonreducing Monosaccharide Moiety of Lipid A "

## BACKGROUND OF THE INVENTION

### (a) Field of the Invention

This invention relates to novel analogs of the nonreducing monosaccharide subunit of lipid A and stereoisomers thereof. These novel compounds have been synthesized by the present inventors in the course of their search for an effective sugar moiety, which exhibits higher biological and immunological activities than natural lipid A.

### (b) Description of the Prior Art

Lipo-polysaccharides are found in the cell-wall of some kinds of gram-negative bacilli as a main component of endotoxin. They exhibit various kinds of biological and immunological activities such as an anti-tumor activity. Lipid A is a lipoid component of lipo-polysaccharides. It is known that the biological and immunological activities of lipo-polysaccharides mostly depend on the lipid A component.

In an attempt to elucidate the chemical structure of lipid A, and to synthesize analogs of the sugar moieties of lipid A which exhibit as many biological and immunological properties of natural lipid A as possible, compounds of the following formula have been described by Galanos and Ludritz et al. in 1977 -[cf. Int. Rev. Biochem. 14: 239 (1977) and Naturwissensch. 65:578 (1987)]

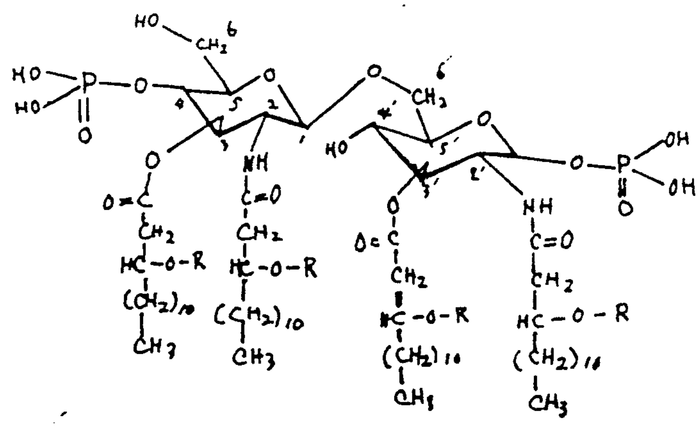

wherein R represents a hydrogen atom or a straight chain aliphatic acid having 12 to 16 carbon atoms, especially myristic acid, that is tetradecanoic acid, represented by a chemical formula of $CH_3(CH_2)_{12}COOH$.

The compounds of the above structure are characterized by two glucosamine groups which are linked at their 1-and 6'-positions and the amino groups are located at the 2-and 2'-positions and the hydroxy groups are located at the 3-and 3'-positions of the glucosamine groups. Moreover, 3-hydroxy-myristic acid residues are attached by an amide or an ester linkage and the phosphoric acid groups are linked to the 1-and 4'-positions, respectively of the glucosamine groups. The compounds thus simultaneously have both hydrophilic and lipophilic substituents on the glucosamine groups.

In the above chemical formula, the left-handed glucosamine group is called the nonreducing subunit.

On the assumption that it is the nonreducing subunit which is mainly responsible for the biological and immunological activities of lipid A, the inventors have carried out an extensive research to synthesize analogs of the nonreducing sugar subunit of lipid A and the thus synthesized products have successively been subjected to a primary biological screening experiment.

SUMMARY OF THE INVENTION

Of the large number of analogs of the nonreducing sugar moiety of lipid A which have been synthesized by the inventors, the compounds represented be the following general formula [I] have been found to have definite biological and immunological activities, for example, inducing interferon-and tumor-necrosis factors:

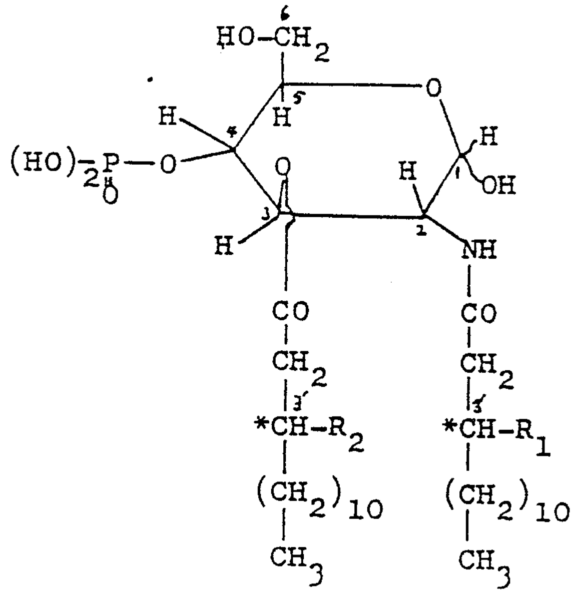

[I]

wherein $R_1$ and $R_2$ are radicals whose definitions are shown in the next table:

Table

| Compound No. | $R_1$ | $R_2$ |
|---|---|---|
| I(R,R) | $-O-CO-(CH_2)_{12}-CH_3$ | $-O-CO-(CH_2)_{12}-CH_3$ |
| II | $-O-CO-(CH_2)_{12}-CH_3$ | -OH |
| II(R,R) | $-O-CO-(CH_2)_{12}-CH_3$ | -OH |
| II(S,S) | $-O-CO-(CH_2)_{12}-CH_3$ | -OH |
| III | -OH | $-O-CO-(CH_2)_{12}-CH_3$ |
| III(R,R) | -OH | $-O-CO-(CH_2)_{12}-CH_3$ |
| III(S,S) | -OH | $-O-CO-(CH_2)_{12}-CH_3$ |
| IV(R) | -H | $-O-CO-(CH_2)_{12}-CH_3$ |

In the foregoing chemical formula and table, the carbon atoms which are marked with an asterisk* indicate an asymmetric carbon atom. Those two asymmetric carbon atoms can have a rectus configuration, hereinafter referred to as (R), or a sinister configuration, hereinafter referred to as (S).

The preparation process of this invention is as follows: reaction of 3'-O-(substituted or non-substituted)-tetradecanoyl radical with the amino group on the C-2 position of a glucopyranose ring is carried out in the presence of dicyclohexylcarbodiimide (DCC) and reaction of the same radical with the hydroxyl group on the C-3 position is carried out in the presence of DCC or dimethylaminopyridine (DMAP). The following reactions are the protection of the hydroxyl groups on the C-4 and C-6 positions of the glucopyranose ring by coupling them with an isopropylidene group and removal thereof, protection of a hydroxyl group on the C-6 position with a trityl group and removal thereof, and reaction of a diphenylphosphono group with the hydroxyl group on the C-4 position and removal of the diphenyl group therefrom.

The inventors were the first who successfully applied these chemical reactions by properly combining them in a suitable order and manner, to the preparation of analogs of the non-reducing monosaccharide moiety of lipid A.

## EXAMPLES

Example 1: Preparation of 2-Deoxy-4-O-phosphono-2-[(3'R)-3'-tetradecanoyloxytetradecanamido]-3-O-[(3'R)-3'-tetradecanoyloxytetradecanoyl]-D-glucose; [Compound No. I (R,R)]

[Step a] Preparation of Benzyl 2-deoxy-4,6-O-iso-propylidene-2-[(3'R)-3'-tetradecanoyloxytetradecanamido-β-D-glucopyranoside; [Introduction of a (3R)-tetradecanoyloxytetradecanoyl group into the C-2 amino group]

Two grams of the known compound benzyl 2-amino-2-deoxy-4,6-O-isopropylidene-β-D-glucopyranoside, of which preparation was published in Agric. Biol. Chem., 48, pages 251 -252 (1984) by some of the inventors et al., were dissolved in anhydrous dichloromethane (20 ml), to which (3R)-3-tetradecanoyloxytetradecanoic acid (3 g) and DCC (2.7 g) were added. The mixture was stirred for 4.5 hours at room temperature and the precipitated DCC-urea was removed by filtration. The remaining solution was washed well with dichloromethane and the filtrate and the washings were combined and concentrated in vacuo. The thus obtained syrups were subjected to column chromatography (Wako gel C-200). The effluents obtained with a mixture of dichloromethane and methanol (400 : 1) were lyophilized from 1,4-dioxane. 2.2 g (91%) of the title compound were obtained.

Melting Point: 66 -70 °C. $[\alpha]_D$ -49.3° (C = 1.127, chloroform).
Analysis (%) for $C_{44}H_{75}NO_8$ = 746.05
Calcd.: C, 70.83; H, 10.13; N, 1.88
Found : C, 70.68; H, 9.99; N, 1.82

[Step b] Preparation of Benzyl 2-deoxy-4,6-O-isopropylidene-2-[(3'R)-3'-tetradecanoyloxytetradecanamide]-3-O-[(3'R)-3'-tetradecanoyloxytetradecanoyl]-β-D-glucopyranoside; [Introduction of a (3R)-tetradecanoyloxytetradecanoyl group into the C-3 hydroxyl group]

The product of the preceding step (1.35 g) was dissolved in anhydrous dichloromethane (9 ml), to which (3R)-tetradecanoyloxytetradecanoic acid (0.82 g), DCC (0.75 g) and DMAP (0.105 g) were added. The mixture was stirred at room temperature. The completion of the reaction was confirmed by means of a thin layer chromatography (ethyl acetate : hexane = 1' : 1). The precipitated urea was removed by filtration and the remaining solution was well washed with dichloromethane. The filtrate and washings were combined and concentrated in vacuo. The thus obtained syrups were subjected to column chromatography (Wako gel C-200). The effluents obtained with a mixture of hexane and ethyl acetate (10 : 1) were lyophilized from 1,4-dioxane. 1.73 g (81 %) of the title compound were obtained.

Melting Point: 64 -65 °C. $[\alpha]_D$ -23.1° (C = 0.995, chloroform).
Analysis (%) for $C_{72}H_{127}NO_{11}$ = 1182.74
Calcd.: C, 73.11; H, 10.82; N, 1.18
Found : C, 73.38; H, 11.00; N, 1.24

[Step c] Preparation of Benzyl 2-deoxy-2-[(3'R)-3'-tetradecanoyloxytetradecanamido]-3-O-[(3'R)-3'-tetradecanoyloxytetradecanoyl]-β-D-glucopyranoside [Removal of the isopropylidene group]

The product of the preceding step (1.36 g) was dissolved in 80 % acetic acid (20 ml) and the mixture was stirred for 3 hours at 45 °C. The reaction mixture was concentrated in vacuo and the residue was subjected to column chromatography (Wako gel C-200). 1.05 g (80 %) of the title compound were obtained after the elution with a mixture of dichloromethane and methanol (100:1).

Melting Point: 101 -101.5 °C. $[\alpha]_D$ -16.8° (C = 0.92, chloroform).

IR$\nu^{nujol}_{max}$ $cm^{-1}$ = 3600 -3200 (OH, NH), 1730 (ester), 1660, 1550 (amido), 760 -690 (ph)

[Step d] Preparation of Benzyl 2-deoxy-2-[(3'R)-3'-tetradecanoyloxytetradecanamido]-3-O-[( 3'R)-3'-tetradecanoyloxytetradenoyl]-6-O-trityl-β-D-glucopyranoside [Tritylation of the C-6 hydroxyl group]

The product of the preceding step (0.87 g) was dissolved in pyridine (10 ml) and was stirred for 3.5 hours at 90°C. The obtained residue was dissolved in chloroform. The solution was washed with 2N hydrochloric acid and then with water and was concentrated in vacuo. The thus obtained syrups were subjected to column chromatography (Wako gel C-200). The effluents obtained with a mixture of dichloromethane and methanol (500 : 1) were lyophilized from 1,4-dioxane. 1.01 g (95%) of the title compound were obtained.

Melting Point: 93 -97°C. $[\alpha]_D$ -19.4° (C = 1.322, chloroform)

[Step e] Preparation of Benzyl 2-deoxy-4-O-diphenylphosphono-2-[( 3'R)-3'-tetradecanoyloxytetradecanamido]-3-O-[( 3'R)-3'-tetradecanoyloxytetradecanoyl]-β-D-glucopyranoside [Introduction of a diphenylphosphono group into the C-4 hydroxyl group and removal of the C-6 trityl group]

The product of the preceding step (0.6 g) was dissolved in a mixed solvent (3 ml) of anhydrous dichloromethane and pyridine (2 : 1), to which DMAP (0.01 g) and diphenylphosphoric acid (0.4 g) were added. The mixture was stirred over night at room temperature. Chloroform was added and the mixture was washed with 2N hydrochloric acid and with water and was then dried over sodium sulfate and concentrated in vacuo. The obtained syrups were subjected to column chromatography (Wako gel C-200). The effluents obtained with dichloromethane were dissolved in acetone (30 ml). $HBF_4$ (0.03 g) was added to the mixture which was stirred for one hour at room temperature. The reaction mixture was neutralized with triethylamine and was concentrated in vacuo. The thus obtained syrups were subjected to column chromatography (Wako gel C-200). The effluents obtained with a mixture of dichloromethane and methanol were lyophilized from 1,4-dioxane. 0.426 g (71%) of the title compound were obtained.

Melting Point: 92 -93°C. $[\alpha]_D$ -17.5° (C = 1.10, chloroform).

NMR data ($CDCl_3$) δ : 3.08 (very broad t, 1H, OH), 3.48 (~d, 1H, $J_{4,5}$ ~10Hz, H-5), 3.5 -3.8 (m, 3H, H-2, H-6), 4.72 (q, 1H, $J_{3,4}$ = $J_{4,5}$ = $J_{4p}$, 9-10Hz, H-4), 5.50 (d, 1H, $J_{1,2}$, 8.4Hz, H-1), 5.56 (dd, 1H, $J_{2,3}$ ~10.3, $J_{3,4}$, [9.2Hz, H-3), 7.1 -7.4 (m, 15H, ph).

[Step f] Preparation of 2-Deoxy-4-O-diphenylphosphono-2-[( 3'R)-3'-tetradecanoyloxytetradecanamido]-3-O-[( 3'R)-3'-tetradecanoyloxytetradecanoyl]-D-glucose [Removal of the C-1 benzyl group]

The product of the preceding step (0.11 g) was dissolved in methanol (20 ml), to which Pd-black (0.05 g), which had been previously reduced, was added. The mixture was stirred over night under hydrogen gas at room temperature. After the removal of the remaining catalyzers by filtration, the filtrate was washed well with methanol. The filtrate and washings were combined and concentrated in vacuo. The obtained syrups were subjected to column chromatography (Wako gel C-200) whereby the column was eluted with a mixture of dichloromethane and methanol (100:1). The title compound (0.1 g) was quantitatively obtained.

Melting Point: 68 -70°C. $[\alpha]_D$ +4.2° (C = 0.622. chloroform).

IR$\nu^{film}_{max}$ $cm^{-1}$ = 3600 -3150 (OH, NH), 1740 (ester), 1660, 1540 (amide), 960 (P-O-ph), 800 -670 - (ph).

NMR data ($CDCl_3$), α:β = Ca. 2 : 1 δ : 0.75 -0.95 (m, 12H, Me), 1.0 -1.7 (m, 84H, $CH_2$), 2.1 -2.5 (m, 8H, CO $\underline{CH_2}$), 4.65 -4.83 (2q, 1H, H-4α,β ), 5.26, (β), 5.46 (α) (2dd, 1H, H-$3_{\alpha,\beta}$ ), 5.33 (d, 2/3H, H-1α), 6.29, 6.83 (2d, 1H, $J_{8,1}$, 6.2Hz, $NH_{\alpha,\beta}$ ), 7.05 -7.4 (m, 10H, ph).

[Step g] The objective compound of Example 1 [ Removal of the diphenyl group from the C-4 diphenylphosphono group]

The product of the preceding step (0.06 g) was dissolved in a mixture (50 ml) of methanol and ethanol - (1 : 1), to which Platinum oxide (0.01 g), which had been previously reduced, was added. The mixture was stirred over night at room temperature under hydrogen gas, After removing the remaining catalyzers by filtration, the reaction mixture was well washed with a mixture of chloroform and methanol (1 : 1). The filtrate and washings were combined and concentrated in vacuo. The thus obtained material was lyophilized from 1,4-dioxane. The objective compound (0.52 g) was quantitatively obtained.

Melting Point: 152 -153°C.

$[\alpha]_D$ +14° (C = 0.52, chloroform : methanol = 3 : 1)

IR $^{KBr}$ $cm^{-1}$ = 3680 -2500 (OH, NH, CH), 1740 (ester), 1660, 1560 (amide).

Analysis (%) for $C_{62}H_{118}NO_{14}P$ = 1132.55

Calcd.: C, 65.75; H, 10.50; N, 1.24

Found : C, 65.39; H, 10.67; N, 1.18

2-Deoxy-4-O-phosphono-2-( 3'-tetradecanoyloxytetradecanmido)-3-O-( 3'-tetradecanoyloxytetradecanoyl)-D-glucose was also prepared by the inventors in the same manner as described in Example 1 except for reacting the C-2 amino and C-3 hydroxyl groups respectively with a 3-tetradecanoyloxytetradecanoyl group (neither a rectus nor a sinister type ). The compound has the following physicochemical constants: $[\alpha]_D$ +11° (C = 0.14, chloroform : methanol = 3 : 1)

Example 2: Preparation of 2-Deoxy-4-O-phosphoryl-2-[( 3'R)-or ( 3'S)-3'-tetradecanoyloxytetradecanamido]-3-O-[( 3'R)-or ( 3'S)-3'-hydroxytetradecanoyl]-D-glucose [Compound No. II (RR) or (SS)]

[Step a] Preparation of (3R)-or (3S)-(benzyloxymethoxy)-tetradecanoic acid [A compound to be introduced into the side chain of the C-3 hydroxyl group]

Respectively 2.3 g of (R)-or (S)-3-hydroxy-tetradecanoic acid acetophenone ester represented by the following formula:

$$CH_3(CH_2)_{10}\underset{OH}{\overset{\star}{C}}HCH_2COOCH_2CO-C_6H_5$$

(* : an asymmetric carbon atom)

(this compound is commercially available) were dissolved in a mixture (18.4 ml) of dichloromethane and diisopropylethylamine (1 : 1).

Benzyloxymethylchloride [= $C_6H_5$-$CH_2OCH_2Cl$, 3.71 ml] was added under cooling with ice, and the mixture then stirred at room temperature. The completion of the reaction was confirmed by means of thin layer chromatography (dichloromethane : methanol = 150 : 1). Methanol was added to the reaction mixture, which was then concentrated in vacuo. The thus obtained residue was dissolved in chloroform and washed with 2N hydrochloric acid and water, dried and concentrated in vacuo. The thus obtained syrups were subjected to column chromatography (Wako gel C-300). The material (a R type or a S type) which was respectively eluded using a mixture of hexane and ethyl acetate (15 : 1) was dissolved in acetic acid (20 ml). Zink powder (4.3 g) was added to the mixture and it was stirred over night at 50°C. The zink powder was removed by filtration and washed with dichloromethane. The filtrate and washings were combined and concentrated in vacuo. The obtained syrups were subjected to column chromatography (Wako gel C-300). The material eluted with dichloromethane or a mixture of hexane and ethyl acetate (10 : 1) was lyophilized from 1,4-dioxane. The title (3R)-or (3S)-compounds were separately obtained (together 2.2 g, 95 %).

(R)-Compound: Syrup $[\alpha]_D$ -6.7° (C = 0.924, chloroform)
Analysis (%) for $C_{22}H_{36}O_4$ = 364.51
Calcd.: C, 72.49; H, 9.96
Found : C, 72.30; H, 10.12
(S)-Compound: Syrup $[\alpha]_D$ +4.0° (C = 1.34, chloroform)
Analysis (%) for $C_{22}H_{36}O_4$
Calcd.: C, 72.49; H, 9.96
Found : C, 72.36; H, 9.89

[Step b] Preparation of (3R)-or (3S)-3-tetradecanoyloxytetradecanoic acid [the compound to be introduced into the C-2 amino group] .

The same (3R)-or (3S)-starting compounds as in the preceding step (together 2.5 g) were separately dissolved in pyridine (27 ml), to which tetradecanoic chloride (= myristoyl chloride, 2.05 g) and a very small quantity of DMAP were added. The mixture was stirred overnight at room temperature. The produced material was dissolved in acetic acid (20 ml) and zink powder (4.3 g) was added thereto. After stirring over night, the zink powder was removed by filtration and well washed with dichloromethane. The filtrate and washings were combined and concentrated in vacuo. The thus obtained syrups were subjected to column chromatography (Wako gel C-300). The material eluted with a mixture of hexane and ethyl acetate (10 : 1) was lyophilized from 1,4-dioxane. The title (3R)-or (3S)-compounds were obtained separately (together 2.7 g, 86 %).

(R)-Compound: Melting Point 38.5 -40°C. $[\alpha]_D$ -0.93° (C = 1.40, chloroform)
Analysis (%) for $C_{28}H_{54}O_4$ = 454.71
Calcd.: C, 73.95; H, 11.97
Found : C, 73.84; H, 12.00

(S)-Compound: $[\alpha]_D$ +0.56° (C = 0.924, chloroform)
Analysis (%) for $C_{28}H_{54}O_4$
Calcd.: C, 73.95; H, 11.97
Found : C, 74.15; H, 11.88

[Step c] Preparation of Benzyl 2-deoxy-4,6-O-isopropylidene-2-[(3'R)-or (3'S)-3'-tetradecanoyloxytetradecanamido]-β-D-glucopyranoside [Introduction of the product of the preceding Step b into the C-2 amino group].

Using the same procedure as in step a of Example 1 and employing as starting material the products ( 3 g) of the preceding step, the title compounds (2.2 g, 91 %; 2.1 g, 89 %) were obtained.

(R)-Compound: Melting Point 66 - 70°C. $[\alpha]_D$ -49.3° (C = 1.127, chloroform)
Analysis (%) for $C_{44}H_{75}NO_8$ = 746.05
Calcd.: C, 70.83; H, 10.13; N, 1.88
Found : C, 70.68; H, 9.99; N, 1.82
(S)-Compound: Melting Point 79 -82°C. $[\alpha]_D$ -44.9° (C = 1.20, chloroform)
Analysis (%) for $C_{44}H_{75}NO_8$
Calcd.: C, 70.83; H, 10.13; N, 1.88
Found : C, 70.60; H, 10.23; N, 1.78

[Step d] Preparation of Benzyl 3-O-[(3'R)-or (3'S)-3'-(benzyloxymethoxy)-tetradecanoyl]-2-deoxy-4,6-O-isopropylidene-2-[(3'R)-or (3'S)-3'-tetradecanoyloxy tetradecanamido]-β-D-glucopyranoside [Introduction of the product of the foregoing step a into the C-3 hydroxyl group]

The product (0.75 g) of the preceding step was dissolved in anhydrous dichloromethane (5 ml), and the product (0.37 g) of the foregoing step b, DCC (0.5 g) and DMAP (0.08 g) were added. The mixture was stirred at room temperature. The completion of the reaction was confirmed by means of thin layer chromatography (ethyl acetate : hexane = 1 : 1). The precipitated urea was removed by filtration and washed with dichloromethane. The filtrate and washings were combined and concentrated in vacuo. The thus obtained syrups were subjected to column chromatography (Wako gel C-200). The effluents obtained with a mixture of hexane and ethyl acetate (10 : 1) were lyophilized from 1,4-dioxane. The title (3'RR)-or (3'SS)-compounds were separately obtained (together 1.08 g, 100 %).

(3'RR)-Compound: Melting Point 71 -72°C. $[\alpha]_D$ -22° (C = 0.91, chloroform)
Analysis (%) for $C_{66}H_{109}NO_{11}$ = 1092.54
Calcd.: C, 72.55; H, 10.06; N, 1.28
Found: C, 72.76; H, 10.20; N, 1.31
(3'SS)-Compound: Melting Point 38 -40°C. $[\alpha]_D$ -32.1° (C = 1.126, chloroform).
Analysis (%) for $C_{66}H_{109}NO_{11}$
Calcd.: C, 72.55; H, 10.06; N, 1.28
Found : C, 72.80; H, 10.31; N, 1.30

[Step e] Preparation of Benzyl 3-O-[(3'R)-or (3'S)-3'-(benzyloxymethoxy)-tetradecanoyl]-2-deoxy-2-[(3'R)-or (3'S)-3'-tetradecanoyloxytetradecanamido]-β-D-glucopyranosid [Removal of the isopropylidene group]

Using the same procedure as in step c of Example 1 and employing as starting material the (3'RR)-or (3'SS)-products (0.87 g, 0.8 g, respectively) of the preceding step, the title compounds were separately obtained (0.73 g, 87 %; 0.66 g, 85%).

(3'RR)-Compound: Melting Point 100 -101.5°C. $[\alpha]_D$-35.9° (C = 0.754, chloroform)
(3'SS)-Compound: Melting Point 94 -96°C. $[\alpha]_D$ -14° (C = 1.213, chloroform)

[Step f] Preparation of Benzyl 3-O-[(3'R)-or (3'S)-3'-(benzyloxymethoxy)-tetradecanoyl]-2-deoxy-2-[(3'R)-or (3'S)-3'-tetradecanoyloxytetradecanamido]-6-O-trityl-β-D-glucopyranoside [Introduction of a trityl group into the C-6 hydroxyl group]

Using the same procedure as in step d of Example 1 and employing as starting material the (3'RR)-or - (3'SS)-products (0.68 g, 0.7 g, respectively) of the preceding step, the title compounds were separately obtained (0.78 g, 93 %; 0.77 g, 90 %).

(3'RR)-Compound: $[\alpha]_D$ -31.2° (C = 0.902, chloroform)
(3'SS)-Compound: Melting Point 70 -72°C. $[\alpha]_D$ -17° (C = 0.87, chloroform)

[Step g] Preparation of Benzyl 3-O-[(3'R)-or (3'S)-3'-(benzyloxymethoxy)-tetradecanoyl]-2-deoxy-4-O-diphenylphosphono-2-[(3'R)-or (3'S)-3'-tetradecanoyloxy tetradecanamido]-β-D-glucopyranoside [Introduction of a diphenylphosphono group into the C-4 hydroxyl group and removal of the C-6 trityl group]

Using the same procedure as in step e of Example 1 and employing as starting material the products - (0.55 g, 0.55 g, respectively) of the preceding step, the title compounds (0.374 g, 68 %; 0.363 g, 66 %) were separately obtained.

(3'RR)-Compound: Melting Point 69.5 -70.5°C. $[\alpha]_D$ -14.5° (C = 0.724, chloroform)

(3'SS)-Compound: Melting Point 67 -70°C. $[\alpha]_D$ -16.3° (C = 1.02, chloroform)

[Step h] Preparation of 2-deoxy-4-O-diphenylphosphono-3-O-[(3'R)-or (3'S)-3'-hydroxytetradecanoyl]-2-[-(3'R)-or (3'S)-3'-tetradecanoyloxytetradecanamido]-D-glucose [Removal of the C-1 benzyl group and removal of a benzyloxymethyl group from the side chain on the C-3 substituent]

Using the same procedure as in step f of Example 1 and employing as starting material the products -(0.158 g, 0.16 g, respectively) of the preceding step, the title compounds (0.128 g, 97 %; 0.14 g, 100 %) were separately obtained.

(3'RR)-Compound: Melting Point 87 -88°C. $[\alpha]_D$ -2.1° (C = 1.17, chloroform)

(3'SS)-Compound: Melting Point 64.5 -65°C. $[\alpha]_D$ +9.5° (C = 0.786, chloroform)

[Step i] Preparation of the objective compound of Example 2 [Removal of the diphenyl group from the C-4 diphenylphosphono group]

Using the same procedure as in step g of Example 1 and employing as starting material the products -(0.128 g, 0.14 g, respectively) of the preceding step, the final objective compounds were separately obtained.

(3'RR)-Compound: Melting Point 172 -174°C. $[\alpha]_D$ +12.8° (C = 0.97, Chloroform : methanol = 3 : 1)

IR$\nu_{max}^{KBr}$ $cm^{-1}$ = 3680 -2500 (OH, NH, CH), 1740, 1720 (ester), 1645, 1650 (amide).

Analysis (%) for $C_{48}H_{92}NO_{13}P$ = 922.21

Calcd.: C, 62.51; H, 10.06; N, 1.52

Found : C, 62.85; H, 9.93; N, 1.60

(3'SS)-Compound: Melting Point 156 -158°C. $[\alpha]_D$ +17.2° (C = 0.571, chloroform : methanol = 3 : 1)

IR$\nu^{KBr}$ $cm^{-1}$ = 3680 -2500 (OH, NH, CH), 1740, 1720 ester), 1655, 1550 (amide).

Analysis (%) for $C_{48}H_{92}NO_{13}P$

Calcd.: C, 62.51; H, 10.06; N, 1.52

Found : C, 62.30; H, 10.26; N, 1.35

2-Deoxy-3-O-(3'-hydroxytetradecanoyl)-4-O-phosphoryl -2-(3'-tetradecanoyloxytetradenamido)-D-glucose was also prepared in the same manner as carried out through the steps from 'a' to 'i' in Example 2 but employing materials which were neither the rectus type nor the sinister type. The compound exhibits the following physico-chemical constants:

$[\alpha]_D$ +8.76° (C = 0.616, chloroform)

UR$\nu_{max}^{NujoL}$ $cm^{-1}$ = 3600 -3200 (OH, NH), 1720 (ester), 1640, 1540 (amide).

Analysis (%) for $C_{48}H_{92}NO_{13}P$

Calcd.: C, 62.51; H, 10.06; N, 1.52

Found : C, 62.39; H, 10.23; N, 1.52

Example 3: Preparation of 2-Deoxy-2-[(3'R)-or (3'S)-3'-hydroxytetradecanamido]-3-O-[(3'R)-or (3'S)-3'-tetradecanoyloxytetradecanoyl]-4-O-phosphoryl-D-glucose

[Step a] Preparation of Benzyl 2-[(3'R)-or (3'S)-3'-(benzyloxymethoxy)tetradecanamido]-2-deoxy-4,6-O-isopropylidene-$\beta$-D-glucopyranoside [Reaction with the C-2 amino group]

Following step c of Example 2 but employing the product of step a of Example 2 for the product of step b of Example 2, the title compounds were obtained.

(3'R)-Compound: Melting Point 109 -110°C, $[\alpha]_D$ -56.5° (C = 0.66, chloroform), Yield 80.2 %

(3'S)-Compound: Melting Point 67 -70°C, $[\alpha]_D$ -49.7° (C = 0.561, chloroform), Yield 84 %

[Step b] Preparation of Benzyl 2-[(3'R)-or (3'S)-3'-(benzyloxymethoxy)tetradecanamido]-2-deoxy-4,6-O-isopropylidene-3-O-[(3'R)-or (3'S)-3'-tetradecanoyloxytetradecanoyl]-$\beta$-D-glucopyranoside [Introduction into C-3 hydroxyl group]

Following step d of Example 2 but employing the product of step a of Example 2 for the product of step b of Example 2, the title compounds were obtained.

(3'RR)-Compound: Melting Point 70 -72°C, $[\alpha]_D$ -24.6° (C = 1.21, chloroform), Yield 92 %.

(3'SS)-Compound: Melting point 39 -40°C, $[\alpha]_D$ -30.1° (C = 1.18, chloroform), Yield 100 %.

[Steps c -g] Preparation of the objective compounds of Example 3

Following steps e -i of Example 2 the product of the preceding step was subjected to the following chemical reactions : (c) Removal of the C-4,6-O-isopropylidene group, (d) Introduction of a trityl group into the C-6 OH group, (e) Introduction of a diphenylphosphono group into the C-4 OH group and removal of the C-6 trityl group, (f) Removal of the C-1 benzyl group and the benzyloxymethyl group from the substituent connected with the C-2 amino group and (g) Removal of the diphenyl group from the substituent connected with the C-4 OH group. Thus, the title compounds were separately obtained.

(3'RR)-Compound: Melting Point 157 -159°C, $[\alpha]_D$ +13.7° (C = 0.512, chloroform : methanol = 3 : 1)
IR$\nu_{max}^{KBr}$ $cm^{-1}$= 3680 -2500 (OH,NH,CH), 1735, 1720 (ester), 1640, 1560 (amide)
(3'SS)-Compound: Melting Point 154 -155°C, $[\alpha]_D$ +18.4° (C = 0.896, chloroform : methanol = 3 : 1)

2-Deoxy-2-(3'-hydroxytetradecanamido)-3-O-(3'-tetradecanoyloxytetradecanoyl)-4-O-phosphoryl-D-glucose was also prepared following the steps from 'a' to 'g' of Example 3 but employing materials which were neither the rectus nor the sinister type. This compound exhibits the following physico-chemical constants:

$[\alpha]_D$ +7.69° (C = 0.442, chloroform)
IR$\nu_{max}^{Nujol}$ $cm^{-1}$= 3600 -3100 (OH, NH), 1720 (ester), 1630, 1540 (amide)
Analysis (%) for $C_{48}H_{92}NO_{13}$ = 922.21
Calcd.: C, 62.51; H, 10.05; N, 1.52
Found : C, 62.44; H, 10.18; N, 1.50

Example 4: Preparation of 2-Deoxy-4-O-phosphoryl-2-tetradecanamido-3-O-[(3'R)-3'-tetradecanoyloxytetradecanoyl]-D-glucopyranose

[Step a] Preparation of Benzyl 2-deoxy-4,6-O-isopropylidene-2-tetradecanamido-β-D-glucopyranoside [Introduction of a tetradecanoyl group into C-2 amino group]

Following step a of Example 1 but employing tetradecanoic acid for (3'R)-3'-tetradecanoyloxytetradecanoic acid, the title compound was obtained in a yield of 88.5 %.

[Step b] Preparation of Benzyl 2-deoxy-2-tetradecanamido-3-O-[(3'R)-3'-tetradecanoyloxytetradecanoyl]-β-D-glucopyranoside [Introduction of a (3R)-3-tetradecanoyloxytetradecanoyl group into the C-3 OH group and removal of the 4,6-O-isopropylidene group]

The product (800 mg) of the preceding step was dissolved in dichloromethane (8 ml), and (3R)-3-tetradecanoyloxytetradecanoic acid (700 mg), DMAP (95 mg) and DCC (400 mg) were added. The mixture was left for 8 hours at room temperature. The completion of the reaction was confirmed by means of thin layer chromatography. The precipitated urea was removed by filtration and the filtrate was concentrated in vacuo. The thus obtained product was dissolved in a mixture of 90 % acetic acid, dichloromethane and methanol and the solution was stirred at 50° C. After confirming the completion of the reaction, the reaction mixture was concentrated in vacuo and the residue was subjected to column chromatograohy (Wako gel C-300). Using as eluent (a) dichloromethane and (b) a mixture of dichloromethane and methanol (250 : 1), the title compound (480 mg, 49 %) was obtained with solvent (b).

$[\alpha]_D$ -21.48° (C = 3.453, chloroform)

[Steps c -f] Preparation of the objective compound of Example 4

Following steps d -g of Example 1 the product of the preceding step was subjected to the chemical reactions of: (c) Tritylation of the C-6 OH group, (d) Diphenylphosphorylation of the C-4 OH groups and removal of the trityl group, (e) Removal of the C-1 benzyl group and (f) Removal of the diphenyl group from the substituent connected to the C-4 group. Thus, the title objective compound was obtained.

Melting Point 150 -151°C.
IR$\nu_{max}^{KBr}$ $cm^{-1}$= 3450 (OH, NH), 2960, 2870 ($CH_2$, $CH_3$), 1740 (ester), 1650, 1560 (amide)

The study of the stereoisomers teaches that a rectus configuration and a sinister configuration, which are formed with an asymmetric carbon atom as a centre, stand in a diastereomer relation to each other, which is not identical with an optical antipode relation between a dextro and a levo type . So, as seen in the examples of this specification, a mother compound and its rectus and sinister compounds exhibit different physico-chemical properties such as melting point, the angle of optical rotation and solubility, and consequently, exhibit different biological and immunological activities with each other.

This is why the inventors were investigating the stereoisomers of some derivatives of the nonreducing monosaccharide subunit of lipid A which have so far been synthesized. They were identified to have certain interesting biological and immunological properties.

To state more concretely, the compounds of this invention are expected to exhibit definite effects for proclotting the enzyme of horseshoe crab, inducing interferon-and tumor-necrosis factors, furthermore, they act as mitogens for polyclonal B cells and as adjuvant.

## Claims

1. Compounds having the general formula I

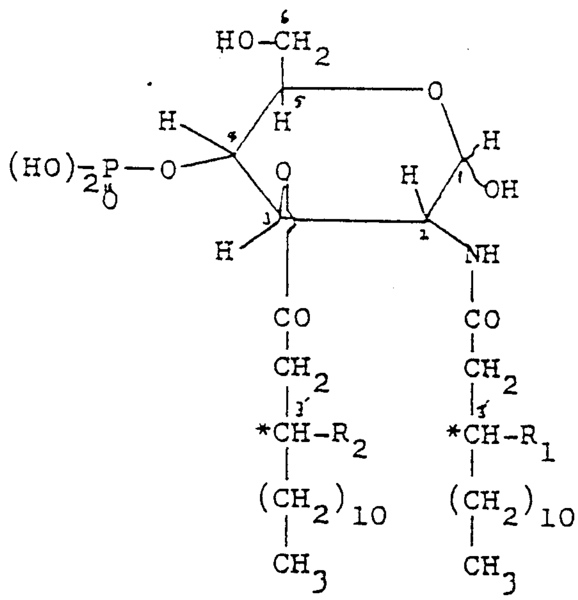

[I]

wherein $R_1$ is a hydrogen atom, hydroxyl group or $-O-CO-(CH_2)_{12}-CH_3$ and $R_2$ represents a hydroxyl group or $-O-CO-(CH_2)_{12}-CH_3$, and stereoisomers thereof.

2. 2-Deoxy-4-O-phosphono-2-[(3'R)-3'-tetradecanoyloxytetradecanamido]-3-O-[(3'R)-3'-tetradecanoyloxytetradecanoyl]-D-glucose

3. 2-Deoxy-4-O-phosphono-2-(3'-tetradecanoyloxytetradecanamido)-3-O-(3'-hydroxytetradecanoyl)-D-glucose

4, 2-Deoxy-4-O-phosphono-2-[(3'R)-3'-tetradecanoyloxytetradecanamido]-3-O-[(3'R)-3'-hydroxytetradecanoyl]-D-glucose

5. 2-Deoxy-4-O-phosphono-2-[(3'S)-3'-tetradecanoyloxytetradecanamido]-3-O-[(3'S)-3'-hydroxytetradecanoyl]-D-glucose

6. 2-Deoxy-4-O-phosphono-2-(3'-hydroxytetradecanamido]-3-O-(3'-tetradecanoyloxytetradecanoyl]-D-glucose

7. 2-Deoxy-4-O-phosphono-2-[(3'R)-3'-hydroxytetradecanamido]-3-O-[(3'R)-3'-tetradecanoyloxytetradecanoyl]-D-glucose

8. 2-Deoxy-4-O-phosphono-2-[(3'S)-3'-hydroxytetradecanamido]-3-O-[(3'S)-3'-tetradecanoyloxytetradecanoyl]-D-glucose

9. 2-Deoxy-4-O-phosphono-2-tetradecanamido]-3-O-[(3'R)-3'-tetradecanoyloxytetradecanoyl]-D-glucose

10. A process for preparing the compounds of the general formula I

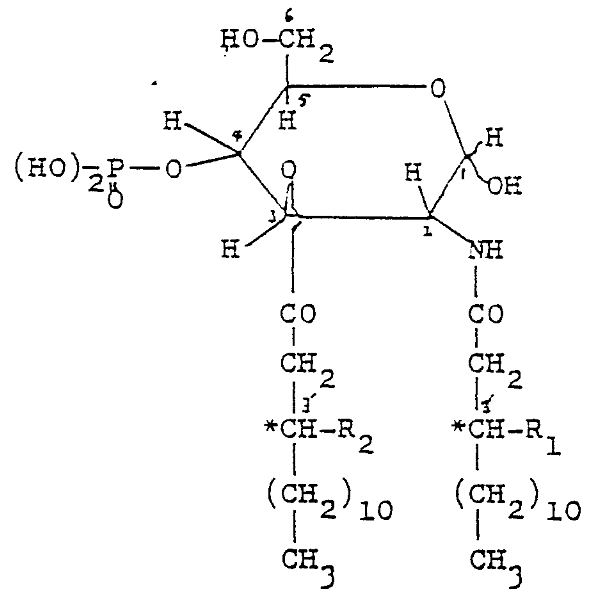

[I]

characterized by

(a) the reaction of a 3'-O-(substituted or non substituted)-tetradecanoyl radical with the amino group in the C-2-position of benzyl 2-amino-2-deoxy-4,6-O-isopropylidene-$\beta$-D-glucopyranoside in the presence of DCC,

(b) reaction of the same radical with the hydroxyl group in the C-3-position in the presence of DCC and/or DMAP,

(c) the removal of the protective groups from the C-4-and C-6-positions,

(d) tritylation of the C-6-hydroxyl group,

(e) introduction of a diphenylphosphono group in the C-4-position and removal of the C-6-tritylgroup,

(f) removal of the C-1-benzyl group,

(g) optional removal of the benzyloxymethyl group from the C-3-side chain, and

(h) removal of the diphenyl group from the C-4-position.

11. The use of the compounds according to claims 1 to 9 for proclotting the enzyme of horseshoe crab, inducing interferon-and tumor-necrosis factors, as mitogens for polyclonal B cells and as adjuvant.